Europäisches Patentamt

European Patent Office

Office européen des brevets

(19)

(11)  EP 0 840 118 B1

(12)  FASCICULE DE BREVET EUROPEEN

(45) Date de publication et mention
de la délivrance du brevet:
**28.11.2001 Bulletin 2001/48**

(51) Int Cl.⁷: **G01N 33/00**

(21) Numéro de dépôt: **97402403.6**

(22) Date de dépôt: **13.10.1997**

(54) **Dispositif de sélection pour fournir à un appareil l'un ou l'autre de deux gaz**

Wahlvorrichtung zum Liefern des einen oder des anderen von zwei Gasen an einer Vorrichtung

Selection device for supplying the one or the other of two gases to an apparatus

(84) Etats contractants désignés:
**BE DE FR GB IT NL SE**

(30) Priorité: **05.11.1996 FR 9613435**

(43) Date de publication de la demande:
**06.05.1998 Bulletin 1998/19**

(73) Titulaire: **L'AIR LIQUIDE, SOCIETE ANONYME POUR
L'ETUDE ET L'EXPLOITATION DES PROCEDES
GEORGES CLAUDE
75321 Paris Cédex 07 (FR)**

(72) Inventeurs:
• **Girard, Jean-Marc
75010 Paris (FR)**

• **Mail, Alain
38570 Theys (FR)**
• **Marot, Yves
78530 Buc (FR)**

(74) Mandataire: **Vesin, Jacques et al
L'AIR LIQUIDE, S.A.,
Service Propriété Industrielle,
75, Quai d'Orsay
75321 Paris Cédex 07 (FR)**

(56) Documents cités:
**FR-A- 2 714 968**

**Description**

L'invention est relative à un dispositif de sélection pour fournir à un appareil, notamment un analyseur de tracés d'impuretés tel qu'un spectromètre de masse à ionisation à pression atmosphérique, un gaz issu d'une de deux lignes d'alimentation.

On connaît, du document FR-A-2714968 au nom de la Demanderesse, un tel dispositif de sélection conçu pour alimenter un analyseur de traces d'impuretés soit avec un gaz à analyser, soit avec un gaz "d'étalonnage" pur ou contenant des impuretés à des concentrations précisément connues. Le dispositif du document précité comprend une première et une deuxième lignes d'alimentation respectivement d'un gaz d'analyse et d'un gaz d'étalonnage. Chaque ligne d'alimentation débouche dans une ligne de purge respective. Les lignes d'alimentation des deux gaz sont raccordées à l'analyseur par l'intermédiaire d'une ligne de raccordement des deux lignes d'alimentation et d'une ligne commune de fourniture de gaz à l'analyseur.

Le contrôle des paramètres d'introduction du gaz dans l'analyseur est assuré d'une part à l'aide d'un régulateur de pression amont monté sur une ligne de décharge reliée en dérivation à la ligne commune de fourniture, et d'autre part, à l'aide d'un régulateur de débit monté sur une ligne de purge additionnelle reliée à une sortie de l'appareil.

Chaque ligne de purge des lignes d'alimentation comporte un débitmètre massique. Ces débitmètres permettent de sélectionner soit le gaz à analyser, soit le gaz d'étalonnage. On comprend alors que pour la sélection du gaz fourni à l'analyseur et pour le contrôle des paramètres d'introduction du gaz dans l'analyseur, on utilise trois débitmètres massiques disposés dans chacune des lignes de purge, ainsi qu'un régulateur de pression amont disposé dans une ligne dérivée de la ligne commune de fourniture de gaz à l'appareil.

La présente invention vise à simplifier le dispositif connu en proposant un dispositif de sélection qui soit plus simple et plus économique.

A cet effet, l'invention a pour objet un dispositif de sélection pour fournir à un appareil l'un de deux gaz, comprenant une première et une deuxième lignes d'alimentation respectivement d'un premier et d'un deuxième gaz, chaque ligne d'alimentation débouchant dans une ligne de purge respective, et une ligne de raccordement commune des deux lignes d'alimentation à une ligne de fourniture commune de gaz reliée à une entrée de l'appareil, une sortie de l'appareil étant reliée à une ligne de purge additionnelle, un organe de création d'une perte de charge étant disposé dans chaque ligne de purge, l'organe de création d'une perte de charge dans la ligne de purge associée à la première ligne d'alimentation ainsi que l'organe de création d'une perte de charge dans la ligne de purge additionnelle étant des organes de régulation de débit, le dispositif se caractérisant en ce que l'organe de création d'une perte de charge dans la ligne de purge associée à la deuxième ligne d'alimentation est un déverseur, et en ce que la ligne de fourniture commune de gaz est uniquement reliée à la ligne de raccordement d'une part, et à l'appareil d'autre part.

D'autres caractéristiques et avantages de l'invention ressortiront de la description suivante donnée à titre d'exemple sans caractère limitatif, en regard des dessins annexés sur lesquels :

La figure 1 est un schéma général d'une installation pour fournir à un appareil, soit un gaz à analyser, soit un gaz pur, ou encore un gaz chargé d'une quantité prédéterminée d'impuretés ;
La figure 2 est une vue en coupe suivant la ligne II/II de la figure 3 d'une vanne du dispositif de régulation de la figure 1 ainsi que d'une vanne d'une unité d'épuration de la figure 1, en position d'isolement;
La figure 3 est une vue en coupe, suivant la ligne III/III de la figure 2, de la même vanne en position de mise en communication;
La figure 4A est une vue en coupe, suivant la ligne IV/IV de la figure 2, de la vanne en position d'isolement;
La figure 4B est une vue en coupe, suivant la ligne IV/IV de la figure 2, de la vanne en position de mise en communication; et
La figure 5 est un schéma d'un étage de dilution.

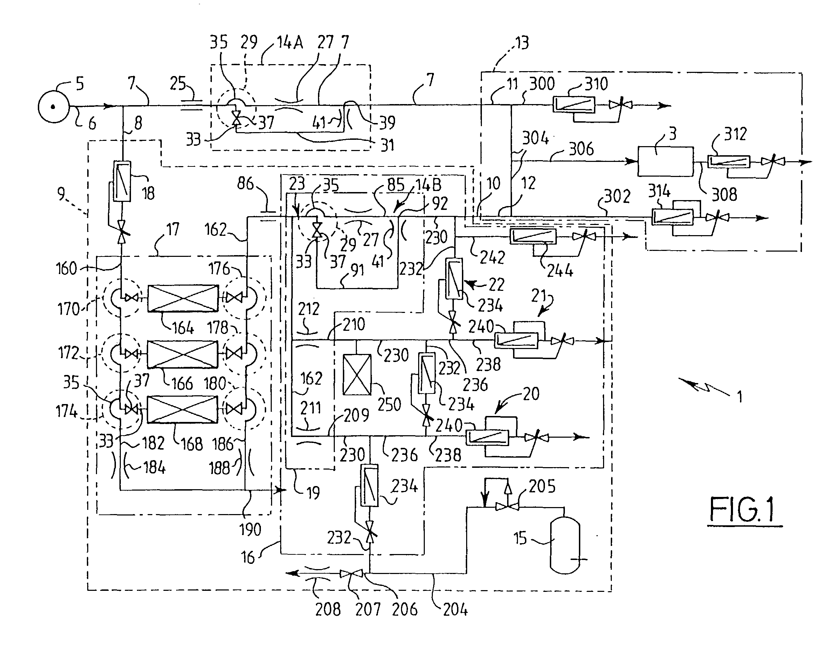

La figure 1 montre une installation 1 de fourniture de gaz à un appareil 3 tel que par exemple un analyseur de traces d'impuretés dans un gaz du type spectromètre de masse à ionisation à pression atmosphérique. Un tel analyseur 3 est capable de mesurer des traces d'impuretés dans un gaz à des concentrations très faibles comprises entre $10^{-2}$ à $10^{-5}$ppm, voire $10^{-3}$ à $10^{-6}$ppm. Comme on va l'expliquer en détail ci-après, cette installation 1 permet de fournir à l'appareil 3 alternativement un gaz pur de référence ou "gaz zéro", c'est-à-dire un gaz contenant typiquement moins de $10^{-4}$ppm d'impuretés, un gaz chargé d'une quantité prédéterminée d'impuretés gazeuses connues telles que par exemple $H_2O$, $CO_2$, $CO$, $O_2$, $CH_4$, $H_2$, etc..., à des concentrations variables dans une plage s'étendant par exemple de $10^{-5}$ppm à $10^{-2}$ppm, ou encore un gaz à analyser. En outre, cette installation doit permettre de contrôler les paramètres d'introduction du gaz dans l'analyseur 3, tels que la pression et le débit.

De plus, cette installation doit permettre l'utilisation successive de différentes sortes de gaz à analyser.

A cet effet, une source 5 d'un gaz sous pression à analyser est reliée par l'intermédiaire d'une ligne de prélèvement 6 à l'installation de fourniture de gaz 1. Cette source 5 comprend par exemple une source unique d'une sorte de gaz à analyser, ou plusieurs sources de pression de différentes sortes de gaz reliées chacune par une ligne de prélèvement à un dispositif destiné à fournir l'un quelconque de plusieurs gaz à un appareil, par exemple tel que le dispositif décrit dans la demande de brevet français FR-A-2.749.924 publiée le 19. 12. 97.

La ligne de prélèvement 6 est reliée d'une part à une ligne d'analyse 7 et d'autre part à une ligne d'alimentation 8 d'un dispositif 9 de fourniture d'un gaz pur ou chargé d'une quantité prédéterminée d'impuretés gazeuses.

La ligne d'analyse 7 et une ligne de sortie 10 du dispositif de fourniture 9 sont reliées chacune à une ligne d'entrée correspondante 11, et 12 d'un dispositif de sélection 13 pour fournir à l'appareil 3 soit le gaz contenu dans la ligne d'analyse 7, soit le gaz débité par la ligne de sortie 10 du dispositif de fourniture 9.

Un dispositif de régulation 14A d'un débit prédéterminé de gaz pour des gaz à analyser ayant des masses molaires sensiblement différentes, est disposé dans la ligne d'analyse 7.

Le dispositif de fourniture 9 comprend une source de gaz pur, une source d'impuretés 15, et des moyens 16 de dilution prédéterminée des impuretés dans le gaz pur.

La source de gaz pur est constituée d'une part par la source 5 du gaz à analyser et, d'autre part, par une unité d'épuration 17 du gaz débité par la source 5, le débit de gaz alimentant l'unité d'épuration étant réglé par l'intermédiaire d'un régulateur de débit massique 18 disposé dans la ligne d'alimentation 8. On notera que l'unité d'épuration se trouve avantageusement en aval de la régulation de débit.

Les moyens de dilution 16 comportent des moyens 19 de division du débit de gaz en sortie de l'unité d'épuration 17, qui alimentent plusieurs étages de dilution 20, 21, 22 disposés en série.

Une branche 23 des moyens 19 de division du débit comporte un dispositif 14B de régulation d'une pression amont prédéterminée de gaz pour des gaz ayant des masses molaires sensiblement différentes, la structure de ce dispositif 14B étant identique à celle du dispositif de régulation 14A.

On va décrire ci-après en détail la structure et le fonctionnement des différentes unités de l'installation de fourniture de gaz 1.

I. <u>Dispositif de régulation de l'écoulement de gaz ayant des masses molaires sensiblement différentes.</u>

<u>I.1. Structure du dispositif de régulation</u>

Le dispositif de régulation 14A est disposé dans la ligne d'analyse 7. En amont de ce dispositif de régulation 14A, est disposée une jauge de pression 25 pour déterminer la pression en amont du dispositif de régulation 14A.

Le dispositif de régulation 14A comporte pour le mode de réalisation représenté, une restriction calibrée 27, par exemple un orifice calibré, disposée dans la ligne d'analyse 7. En amont de l'orifice 27 est disposée une vanne de branchement 29, représentée de façon schématique entourée par des tirets, pour la mise en service sélective d'une ligne de dérivation 31.

La vanne 29 comprend un premier conduit 33 relié en permanence par une extrémité à la ligne de dérivation 31. Elle comprend en outre un deuxième conduit 35 toujours ouvert qui est disposé dans la ligne d'analyse 7.

Le premier conduit 33 et le deuxième conduit 35 de la vanne 29 peuvent être mis en communication par l'intermédiaire d'un actionneur 37, comme cela sera expliqué en détail ci-après, commutable entre une position de mise en communication du premier conduit 33 avec le deuxième conduit 35, et une position d'isolement du premier conduit 33 par rapport au deuxième conduit 35. La ligne de dérivation 31 est reliée par son autre extrémité 39, en aval de l'orifice 27, à la ligne d'analyse 7.

Un deuxième orifice calibré 41 est préférentiellement disposé dans la ligne de dérivation 31, au plus près de l'extrémité 39 de celle-ci. Ainsi, un volume de stagnation d'écoulement, formé en position d'isolement des deux conduits 33 et 35 par la partie de la ligne de dérivation 31 située entre l'orifice 41 et l'extrémité 39, est le plus petit possible.

La structure du dispositif de régulation 14B est identique à celle du dispositif 14A. C'est pourquoi les éléments identiques portent les mêmes numéros de référence.

Ainsi, ce dispositif comporte un premier orifice 27 disposé dans une ligne 85 d'alimentation d'un gaz pur débité en sortie de l'unité d'épuration 17. Dans cette ligne 85 est disposée une vanne de branchement 29 identique à celle du dispositif 14A. Au conduit 33 de la vanne 29 est reliée une extrémité d'une ligne de dérivation 91. L'autre extrémité de cette ligne 91 est raccordée en aval de l'orifice 27 à la ligne d'alimentation 85. Un orifice calibré 41 est disposé au plus près de l'extrémité 92 par laquelle la ligne de dérivation 91 est raccordée à la ligne d'alimentation 85.

<u>I.2. Structure de la vanne des dispositifs de régulation :</u>

On décrit ci-après en détail un exemple de réalisation de la vanne 29 installée dans les dispositifs de régulation 14A et 14B. Une telle vanne, du type DAD électropolie, est par exemple dérivée d'une vanne commercialisée par la société NUPRO et fabriquée par la société SWAGELOK.

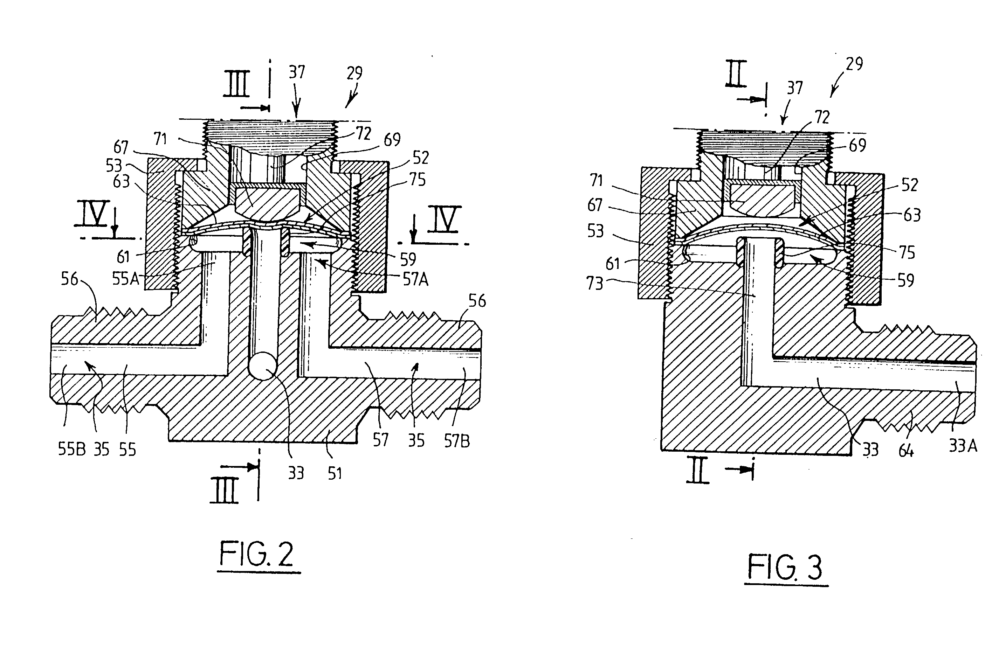

Comme cela est représenté sur les figures 2 et 3, la vanne 29 comporte un corps 51 dans lequel sont réalisés le premier conduit 33 et le deuxième conduit 35, un obturateur 52 et un actionneur 37, représenté en partie, vissé sur le corps 51 au moyen d'un écrou 53.

Le deuxième conduit 35 (Fig.2) est formé par deux tronçons de conduit 55 et 57 et par une chambre annulaire 59, à symétrie de révolution. Dans une partie latérale du fond de cette chambre 59 débouche l'une 55A, 57A des deux extrémités de chaque tronçon de conduit 55, 57.

L'autre extrémité 55B, 57B de chaque tronçon de conduit 55, 57 débouche dans un raccord respectif latéral 56 du corps 51. Ces extrémités 55B et 57B sont diamétralement opposées. Les deux raccords 56 sont destinés à être reliés à la ligne d'analyse 7 en ce qui concerne le dispositif de régulation 14A et à ligne d'alimentation 85 en ce qui concerne le dispositif de régulation 14B.

La chambre 59 est formée par un évidement 61 sensiblement cylindrique, ménagé dans la face supérieure du corps 51, et par l'obturateur 52. Cet obturateur est lui-même constitué d'un ensemble de deux membranes accolées 63 qui recouvrent l'évidement 61 et constituent la paroi supérieure de la chambre 59.

Les membranes 63 sont réalisées en un matériau élastiquement déformable, par exemple du métal. Chaque membrane 63 est un disque dont la partie centrale est bombée dans une direction opposée du corps 51. Le bord des membranes 63 est serré de façon étanche entre le bord annulaire de l'évidement 61 et un bord annulaire d'une pièce de maintien 67 qui fait partie de l'actionneur 37. La pièce 67 est réalisée en forme de cuvette afin de permettre un débattement de la partie bombée des membranes 63.

La pièce de maintien 67 comporte dans sa partie centrale, en regard des membranes 63, un alésage 69 de guidage dans lequel peut coulisser un poussoir 71 mû par une tige 72 de l'actionneur 37.

Le premier conduit 33 de la vanne 29 comprend un simple perçage borgne droit qui s'étend perpendiculairement à l'axe défini par les extrémités 55B, 57B des tronçons de conduit 55, 57 et une cheminée de raccordement 73 qui débouche au centre de l'évidement 61.

Une extrémité 33A, du premier conduit 33 débouche dans un raccord latéral respectif 64 du corps 51, et est destinée à être reliée à la ligne de dérivation 31 dans le cas du dispositif 14A ou à la ligne de dérivation 91 dans le cas du dispositif 14B.

L'extrémité de la cheminée 73 débouchant dans l'évidement 61 comporte un joint d'étanchéité cylindrique 75 emmanché à force dans le corps 51 de la vanne et qui fait saillie dans la chambre 59.

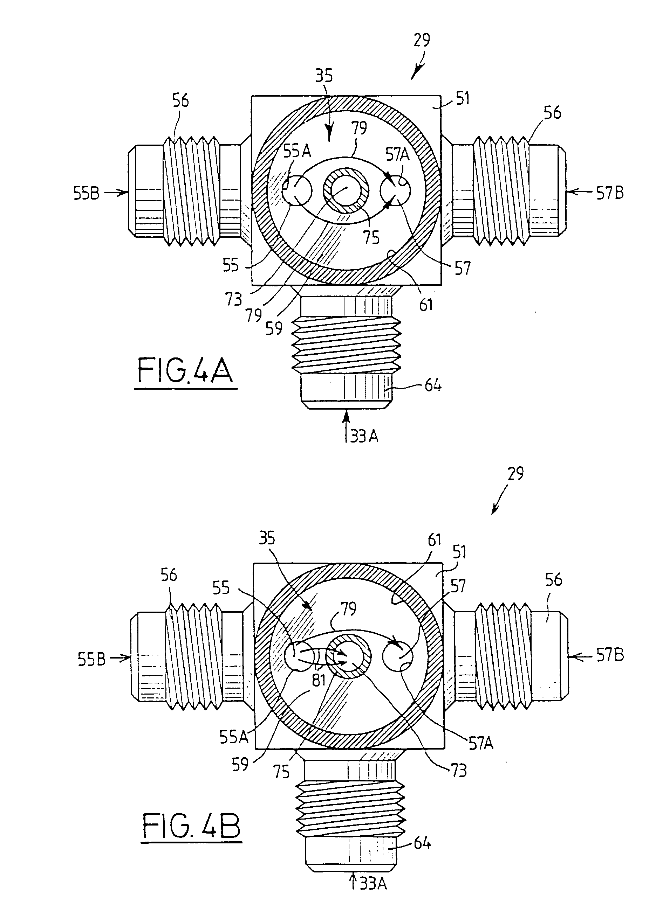

Les figures 2 et 4A montrent la vanne 29 en position d'isolement du premier conduit 33 par rapport au deuxième conduit 35. Dans un tel cas, la partie centrale des membranes 63 est pressée de façon étanche par le poussoir 71 sur le joint d'étanchéité 75, de sorte que la cheminée 73 est isolée par rapport à la chambre 59.

Un gaz introduit dans le deuxième conduit 35 de la vanne circule néanmoins librement, par exemple du tronçon de conduit 55 dans la chambre 59 et, ensuite, dans le tronçon de conduit 57 comme cela est représenté par les flèches 79 sur la figure 4A. On comprend que le deuxième conduit 35 de la vanne ainsi formée ne présente aucun volume de stagnation d'écoulement.

La figure 3 et la figure 4B correspondent à une position de mise en communication du premier conduit 33 avec le deuxième conduit 35. Dans un tel cas, le poussoir 71 est en retrait. Par la force d'élasticité des membranes 63, celles-ci reprennent leur forme initiale bombée. Par conséquent, il se forme un espace libre entre les membranes 63 et le joint d'étanchéité 75, de sorte que le gaz s'écoulant dans le deuxième conduit 35 se déverse en grande partie via la cheminée 73 dans le premier conduit 33 comme cela est représenté par les flèches 81 sur la figure 4B.

I.3. <u>Fonctionnement du dispositif de régulation</u> :

On explique ci-après le fonctionnement des dispositifs de régulation 14A, 14B en regard de la figure 1. A cet effet, on distingue deux modes de fonctionnement.

Dans un premier mode de fonctionnement, la pression en amont du dispositif de régulation est imposée, et le débit doit être à peu près le même pour deux gaz ayant des masses molaires sensiblement différentes. Ce mode de fonctionnement correspond au dispositif 14A disposé dans la ligne d'analyse 7.

Dans un deuxième mode de fonctionnement, le débit de gaz en amont du dispositif de régulation est imposé, et on veut assurer un régime d'écoulement sonique ou proche du régime sonique. Ce mode de fonctionnement correspond à celui qui est réalisé par le dispositif de régulation 14B.

I.3.1. Fonctionnement à pression imposée :

Pour un gaz léger, tel que par exemple l'hydrogène, on commute la vanne 29 du dispositif de régulation 14A dans la position d'isolement du premier conduit 33 par rapport au deuxième conduit 35. Le gaz de la source 5 s'écoule librement dans la ligne d'analyse 7 à travers le deuxième conduit 35 et l'orifice 27. Pour un écoulement en régime sonique, c'est-à-dire dans le cas où le rapport entre la pression amont de l'orifice 27 et la pression aval de cet orifice est supérieur à 2, on sait que le débit volumique $D_{27}$ du gaz à travers l'orifice 27 est égale à :

$$D_{27} = K \times P \times S_{27} \times M^{-1/2}$$

avec :

P = pression en amont de l'orifice 27,
$S_{27}$ = section de l'orifice 27,
M = masse molaire du gaz s'écoulant à travers l'orifice, et
K = constante qui dépend de la température et de la nature des gaz.

Grâce au fait que le conduit 35 de la vanne 29 ne présente pas de volume de stagnation d'écoulement et que l'orifice 41 de la ligne de dérivation 31 est disposé près de l'extrémité 39 de cette ligne, le dispositif de régulation 14A n'introduit qu'un volume de stagnation d'écoulement négligeable dans la ligne d'analyse 7 en position d'isolement du premier conduit 33 par rapport au deuxième conduit 35 de la vanne.

Dans le cas d'un gaz, tel que par exemple $N_2$, ayant une masse molaire plus importante, à pression égale, le débit à travers l'orifice 27 chute proportionnellement à la racine carrée du rapport des masses molaires des deux gaz, par exemple pour $N_2$ et $H_2$, à environ un quart du débit obtenu pour $H_2$. Pour maintenir dans ce cas le débit fourni par la ligne d'analyse 7 à peu près au même niveau que le débit d'un gaz de masse molaire faible, la vanne 29 est commutée à l'état de mise en communication du premier conduit 33 avec le deuxième conduit 35. Le gaz s'écoule alors non seulement à travers l'orifice 27, mais aussi dans la ligne de dérivation 31 à travers l'orifice 41.

Au débit $D_{27}$ s'ajoute alors, au raccordement des lignes d'analyse 7 et de dérivation 31, le débit $D_{41}$ qui est donné, en régime sonique, par la relation :

$$D_{41} = K \times P \times S_{41} \times M^{-1/2}.$$

La nomenclature utilisée est analogue à celle utilisée pour le débit $D_{27}$.

Afin que le débit régulé par le dispositif de régulation 14A soit à peu près égal pour deux gaz ayant respectivement une masse molaire $M_1$ et $M_2$, on choisit la section $S_{41}$ de l'orifice 41 de telle sorte qu'elle satisfait la relation :

$$S_{41} = ((M_1^{1/2} / M_2^{1/2}) \times S_{27}) - S_{27}$$

où $M_1$ est la masse molaire d'un gaz ayant une masse molaire importante, et
$M_2$ est la masse molaire d'un gaz ayant une masse molaire faible.

De préférence, on choisit la section $S_{41}$ de l'orifice 41 de telle sorte que le débit de $H_2$ à l'état d'isolement de la vanne 29 soit comparable au débit de $N_2$ à travers l'orifice 27 et l'orifice 41 à l'état de mise en communication de la vanne 29.

I.3.2. Fonctionnement à débit imposé :

Ce mode de fonctionnement du dispositif de régulation est tout particulièrement intéressant dans le cas où le débit en amont du dispositif de régulation est imposé, et où il faut assurer un régime sonique ou proche du régime sonique pour des orifices de régulation de débit, tels que par exemple ceux des moyens de division de débit 19. Le débit de gaz en amont du dispositif de régulation 14B est imposé par le régulateur de débit 18.

On comprendra ici que le régime « sonique » est préféré et que le fait de s'en éloigner trop rend tout simplement difficile le calcul de la répartition des débits. On s'attachera alors à rechercher un rapport des débits amont et aval se situant entre 1,5 et 30.

Pour expliquer le fonctionnement à débit imposé, on ne considère que le dispositif de régulation 14B seul disposé dans la ligne d'alimentation 85, comme cela a été décrit au sujet du dispositif 14A. Dans le cas où le dispositif 14B est disposé dans les moyens 19 de division de débit, le raisonnement présenté ci-après s'applique de manière correspondante.

A débit volumique imposé, la pression en amont de l'orifice 27 en position d'isolement de la vanne 29 est donnée par la relation :

$$P = K' \times D_{18} \times M^{1/2} \times S_{83}^{-1}$$

avec :

$D_{18}$ = débit volumique imposé par le régulateur de débit 18,
K' = une constante qui dépend de la nature du gaz et de la température,
$S_{27}$ = la section de l'orifice 27.

La section de l'orifice 27 est dimensionnée de telle manière que pour un gaz léger tel que $H_2$, on atteint des conditions soniques en amont de l'orifice 27.

Dans le cas d'un gaz, tel que $N_2$, ayant une masse molaire importante, à débit égal, la pression en amont de l'orifice 27 est environ quatre fois supérieure par rapport à la pression amont obtenue pour $H_2$.

C'est pourquoi on commute la vanne 29 à l'état de mise en communication du premier conduit 33 avec le deuxième conduit 35. Le gaz s'écoule alors non seulement à travers l'orifice 27, mais aussi à travers l'orifice 41. La pression en amont de l'orifice 27 ainsi que de l'orifice 41 est donnée par la relation

$$P = K' \times D_{18} \times M^{1/2} \times (S_{27} + S_{41})^{-1}$$

avec :

$S_{41}$ = section de l'orifice 41.

Pour que la pression en amont du dispositif de régulation 14B atteigne les conditions soniques pour deux gaz de masse molaire différente, on choisit la section $S_{41}$ de l'orifice 41 de telle sorte qu'elle vérifie la relation

$$S_{41} = ((M_1^{1/2} / M_2^{1/2}) \times S_{27}) - S_{27}$$

où $M_1$ est la masse molaire d'un gaz de masse molaire importante, et $M_2$ est la masse molaire d'un gaz ayant une masse molaire faible.

Bien entendu, on peut utiliser au lieu des orifices 27 et 41 des restrictions calibrées quelconques telles que par exemple des capillaires, ou encore des frittés.

Par ailleurs, on prévoit l'utilisation de vannes 29 à membranes qui comportent des moyens de commande de l'actionneur 37 entre les positions de mise en communication et d'isolement, telles que des vannes pneumatiques ou des vannes à commande électromagnétique. Les moyens de commande du déplacement de l'actionneur de la vanne sont alors reliés à une unité de commande, telle que par exemple un micro-ordinateur ou un automate de commande.

II.Dispositif de fourniture d'un gaz pur chargé d'une quantité prédéterminée d'impuretés gazeuses.

On présente ci-après en détail les différentes unités du dispositif de fourniture 9 d'un gaz pur chargé d'une quantité prédéterminée d'impuretés gazeuses, à savoir l'unité d'épuration 17, la source d'impuretés 15 et les moyens 16 de dilution prédéterminée des impuretés dans le gaz pur.

II.1. L'unité d'épuration :

II.1.1. Structure de l'unité d'épuration

L'unité d'épuration 17 comprend une ligne d'entrée 160 et une ligne 162 de fourniture d'un gaz pur, entre lesquelles sont disposés en parallèle trois épurateurs 164, 166, 168, tels que par exemple un épurateur d'azote, un épurateur d'hydrogène et un épurateur d'argon et hélium.

L'entrée de chaque épurateur est raccordée par l'intermédiaire d'une vanne de branchement 170, 172, 174 à la ligne d'entrée 160. La sortie de chaque épurateur 164, 166, 168 est reliée à la ligne de fourniture 162 de l'unité d'épuration 17 au moyen d'une vanne 176, 178, 180 de mise en circuit du gaz épuré.

Les vannes 170 à 180 sont identiques aux vannes 29 des dispositifs de régulation 14A, 14B.

Ainsi, chaque vanne de branchement 170, 172, 174 ainsi que chaque vanne 176, 178, 180 de mise en circuit du gaz épuré comprend un premier conduit 33 relié en permanence par une extrémité à un épurateur correspondant 164, 166, 168. Les deuxièmes conduits 35 des vannes de branchement 170, 172, 174 sont disposés dans la ligne d'entrée 160. Les deuxièmes conduits 35 des vannes 176, 178, 180 de mise en circuit du gaz pur sont disposés dans la ligne de fourniture 162.

La ligne d'alimentation 160 est reliée, en aval de la vanne 174, à une ligne de purge 182 dans laquelle est disposé un organe 184 de création d'une perte de charge, tel qu'un orifice calibré.

La ligne de sortie 162 est reliée, à l'extrémité opposée aux moyens 16 de dilution, à une ligne de purge associée 186 dans laquelle est disposé un organe de création d'une perte de charge 188, tel qu'un orifice calibré.

Les lignes de purge 182 et 186 sont réunies en une ligne de purge commune 190 en aval des orifices calibrés 184 et 188.

II.1.2. Fonctionnement de l'unité d'épuration

En fonction de la sorte de gaz qui est débitée par la source 5, on commute par exemple la vanne d'entrée 170 de l'épurateur 164 associé à ce gaz ainsi que la vanne de sortie correspondante 176 à l'état de mise en communication des conduits 33 et 35. Le gaz à analyser de la source 5 circule librement à travers l'épurateur 164 qui purifie ce gaz. Les autres vannes 172, 174, 178 et 180 sont à l'état d'isolement.

Si on change la sorte de gaz débité par la source de pression 5, on commute les vannes 170, 176 jusqu'alors ouvertes à l'état d'isolement et on va commuter les vannes associées à un autre épurateur à l'état de mise en communication.

Grâce à la construction des vannes 170 à 180 et des orifices permettant un débit de fuite, les lignes d'alimentation 160 et de sortie 162 sont purgées en permanence. Cette unité d'épuration 23 présente en outre l'avantage qu'elle puisse être utilisée avec différentes sortes de gaz à épurer et qu'elle soit pratiquement libre de volumes de stagnation d'écoulement.

Avantageusement, pour la commutation des vannes, on utilise des vannes à membranes qui comportent des moyens de commande de la commutation de l'actionneur entre les positions de mise en communication et d'isolement, telle que par exemple des vannes pneumatiques ou des vannes à commande électromagnétique. Les moyens de commande du déplacement de l'actionneur de chaque vanne sont reliés à une unité de commande telle que par exemple un micro-ordinateur ou encore un automate logique. Cette unité de commande comporte des moyens logiques de commutation. Ces moyens logiques sont réalisés, par exemple, par un programme informatique chargé dans le micro-ordinateur, qui exclut que deux épurateurs puissent être simultanément en communication avec la ligne d'alimentation 160 et la ligne de sortie 162.

II.2. <u>La source d'impuretés</u>

La source d'impuretés 15 comprend un réservoir contenant un mélange de différentes sortes de gaz telles que par exemple $N_2$, $CO_2$, CO, $O_2$, $CH_4$, $H_2$, Ar, Kr, Xe, He, etc... Ce mélange a été réalisé de telle sorte que les concentrations volumiques de la majorité des gaz de ce mélange sont du même ordre de grandeur.

Pour des raisons de sécurité, la concentration d'un gaz inerte, tel que par exemple de l'hélium, est choisie préférentiellement beaucoup plus élevée que la concentration volumique de tous les autres constituants du mélange. Grâce à une telle composition du mélange, des oxydants, tels que $O_2$ ou CO, peuvent coexister ensemble avec des combustibles, tels que par exemple $CH_4$, sans qu'il y ait un risque d'inflammabilité ou d'explosion du réservoir 15.

Afin de connaître précisément, après dilution, le contenu du gaz pur en traces d'impuretés, la composition du mélange du réservoir 15 a été déterminée préalablement avec précision à l'aide de moyens d'analyse des gaz, comme par exemple un chromatographe de phases gazeuses. Le réservoir 15 peut être par exemple une bouteille sous haute pression (typiquement 200 bars).

Les gaz du mélange sont prélevés ou introduits dans les moyens de dilution 16 par l'intermédiaire d'une ligne de prélèvement 204 dans laquelle est placé un régulateur de pression aval 205. La ligne de prélèvement 204 débouche dans une ligne de purge 206 associée dans laquelle sont placés une vanne d'arrêt 207 et un organe de création d'une perte de charge, tel qu'un orifice calibré 208.

II.3. <u>Les moyens de dilution prédéterminée de l'impureté gazeuse dans le gaz pur.</u>

Les moyens de dilution 16 comprennent d'une part les moyens 19 de division du débit du gaz pur délivré par la ligne d'alimentation 162 en sortie de l'unité d'épuration 17, et, d'autre part, trois étages de dilution 20, 21, 22 disposés en série. Une jauge de pression 86 est disposée dans la ligne 162 en amont des moyens de division.

Les moyens de division 19 comprennent le dispositif de régulation 14B et deux lignes 209, 210, reliés en parallèle à la ligne d'alimentation 162. Dans chaque ligne 209, 210 est disposée une restriction calibrée 211, 212 telle qu'un orifice calibré.

La ligne du dispositif de régulation 14B ainsi que les lignes 209 et 210 avec leurs orifices 211, 212 respectifs, forment chacun une branche d'alimentation en gaz pur d'un étage de dilution 20, 21, 22 correspondant.

Le fonctionnement de tels moyens de division de débit est décrit en détail dans la demande de brevet FR-A-2714968 au nom de la Demanderesse. C'est pourquoi ce fonctionnement ne sera pas décrit ci-après.

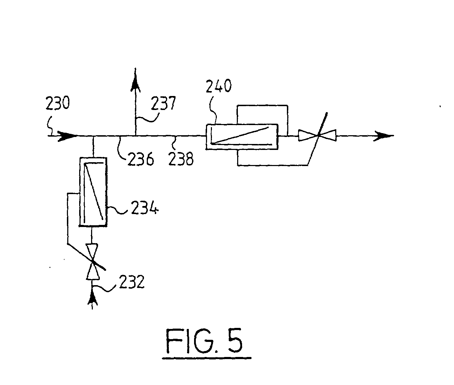

La figure 5 montre un exemple d'un étage de dilution 20, 21 ou 22. Un étage de dilution 20, 21 ou 22 comprend une ligne 230 d'alimentation en gaz pur reliée à une branche correspondante des moyens de division 19, et une ligne 232 d'alimentation en gaz contenant des impuretés.

Un régulateur de débit massique 234 est disposé dans la ligne d'alimentation 232 afin de permettre de faire varier le taux de dilution de l'étage de dilution.

Les lignes d'alimentation 230 et 232 sont raccordées ensemble de telle sorte qu'elles débouchent dans une ligne commune 236 de mélange. La ligne 236 débouche d'une part dans une ligne de sortie 237 de l'étage de dilution. Cette ligne de sortie 237 est reliée à la ligne d'alimentation 232 de l'étage de dilution placé juste en aval.

D'autre part, dans le cas des étages de dilution 20 et 21, la ligne de dilution 236 est reliée à une ligne de purge 238 dans laquelle est disposé un déverseur 240. On règle la pression d'un déverseur dans un étage de dilution de telle sorte que, d'une part, les conditions soniques pour les moyens de dilution sont préférentiellement respectées, et que, d'autre part, la pression réglée est un peu supérieure à la pression qui est réglée dans le déverseur placé dans un étage de dilution en aval, afin d'assurer un écoulement des gaz en direction de l'analyseur 3.

La ligne d'alimentation 232 de l'étage de dilution 20 est raccordée, en aval du régulateur de pression 205 et en amont de la vanne d'arrêt 207, à la ligne de prélèvement 204.

La ligne de sortie 237 du dernier étage de dilution 22 débouche dans la ligne de sortie 10 du dispositif de fourniture 9, en aval du dispositif de régulation 14B.

Les débits à l'entrée de chaque étage de dilution sont réglés de telle façon qu'on obtient à peu près une dilution de 1/1000 du gaz débité par la ligne 232 dans le gaz pur débité par la ligne 230.

Bien entendu, la quantité "zéro" d'impureté est également une quantité prédéterminée de traces d'impuretés que le dispositif doit fournir à l'analyseur 3. C'est pourquoi la ligne d'alimentation 232 du dernier étage de dilution 22 comporte une ligne de dérivation 242 dans laquelle est placé un régulateur de débit massique 244.

La ligne de sortie 10 du dispositif de fourniture 9 débite un gaz pur dans le cas où on règle le régulateur 244 sur un débit supérieur à celui du régulateur 234 de l'étage de dilution 22, et elle débite un gaz pur chargé d'une quantité prédéterminée de traces d'impuretés dans le cas où le débit réglé par le régulateur 244 est inférieur à celui du régulateur 234.

La génération de l'impureté $H_2O$ est réalisée par exemple par une cartouche de perméation 250 débitant environ 250ng/mn et reliée en dérivation à la ligne de gaz pur 230 du deuxième étage de dilution 21.

Cette cartouche de perméation contient de l'eau chauffée ici à environ 50 °C et comporte à une extrémité une membrane de silicone à travers laquelle la molécule $H_2O$ diffuse.

On notera que la cartouche pourrait être placée sur le premier étage pour un taux de perméation plus élevé, le taux de perméation étant choisi pour générer sur la ligne une teneur équivalente à celle des impuretés gazeuses présentes à ce niveau de dilution.

Le tableau 1 ci-dessous montre, pour une composition préalablement déterminée du mélange de gaz dans le réservoir 15, deux exemples de fabrication d'un gaz pur chargé d'une quantité prédéterminée de traces d'impuretés, débité par la ligne de sortie 10 du dispositif de fourniture 9, réalisés une fois avec l'hydrogène comme gaz pur et une fois avec l'azote comme gaz pur.

Tableau 1

| Impuretés | Composition du réservoir en % volumiques | Concentration des impuretés gaz pur $H_2$ | Concentration des impuretés gaz pur $N_2$ |
|---|---|---|---|
| $O_2$ | 5% | 0,20ppb | 1,62ppb |
| $H_2$ | 5% | - | 1,62ppb |
| $N_2$ | 5% | 0,20ppb | - |
| Ar | 5 % | 0,20ppb | 1,62ppb |
| CO | 5% | 0,20ppb | 1,62ppb |
| $CO_2$ | 5% | 0,20ppb | 1,62ppb |
| $CH_4$ | 5% | 0,20ppb | 1,62ppb |
| Xe | 5% | 0,20ppb | 1,62ppb |
| Kr | 5% | 0,20ppb | 1,62ppb |
| He | 55% | 2,19ppb | 17,85ppb |
| $H_2O$ | cartouche de perméation | 0,81ppb | 1,58ppb |

Grâce au fait que la composition initiale du réservoir 15 a été déterminée avec précision au préalable et que ces impuretés sont diluées d'une manière très précise, on obtient un gaz pur contenant une concentration précisément connue des traces d'impuretés.

De plus, à l'aide des débitmètres 234 dans la ligne d'alimentation 232 de chaque étage de dilution 20, 21, 22, on peut faire varier la plage des concentrations des traces d'impuretés d'un facteur 100.

Grâce au fait que le réservoir 15 contient un mélange de plusieurs gaz, celui-ci constitue des traces d'impuretés dans différents gaz purs. Ensemble avec l'unité d'épuration 17, la génération d'un gaz pur chargé de quantités prédéterminées de traces d'impuretés données est alors considérablement facilitée par ce dispositif.

III. <u>Dispositif de sélection d'un de deux gaz</u> :

Comme cela a déjà été décrit, le dispositif de sélection d'un de deux gaz 13 comprend deux lignes d'alimentation 11, 12 dont une 11 est reliée à la ligne d'analyse 7 et l'autre 12 à la ligne de sortie 10 du dispositif de fourniture 9.

Chaque ligne d'alimentation 11, 12 débouche dans une ligne de purge 300 et 302 respective. Les lignes d'alimentation 11 et 12 sont reliées ensemble par l'intermédiaire d'une ligne de raccordement 304. La ligne de raccordement 304 est reliée à l'analyseur 3 par l'intermédiaire d'une ligne de fourniture de gaz 306. La sortie de l'analyseur 3 débouche également dans une ligne de purge 308 associée. Les lignes de purge 300 et 308 comportent chacune un régulateur de débit massique 310, 312. Dans la ligne de purge 302 est disposé un déverseur 314.

Afin de fournir à l'analyseur 3 le gaz contenu dans la ligne 11, on règle la somme des débits $D_{310} + D_{312}$ réglés par les régulateurs de débit 310 et 312 sur un débit inférieur au débit $D_{11}$ débité par la ligne 11. Pour fournir à l'analyseur 3 un gaz d'étalonnage, c'est-à-dire soit un gaz pur, soit un gaz chargé d'une quantité prédéterminée d'impuretés, on règle le débit $D_{310}$ du régulateur de débit 310 pour qu'il soit supérieur au débit $D_{11}$ venant de la ligne 11, et le débit $D_{312}$ réglé par le régulateur de débit 312 pour qu'il soit un peu inférieur au débit de gaz $D_{12}$ de la ligne 12. La pression du gaz introduit dans l'analyseur 3 est réglée par le déverseur 314 disposé dans la ligne de purge 302.

Avantageusement, par un tel agencement, on peut non seulement sélectionner la fourniture à l'analyseur 3 du gaz à analyser ou du gaz d'étalonnage, mais on peut aussi régler des paramètres d'introduction des gaz dans l'analyseur 3 tels que le débit et la pression.

Grâce au fait que l'on utilise un nombre inférieur d'éléments de régulation par rapport au dispositif décrit dans la demande de brevet FR-A-2714968 précitée, on diminue considérablement le coût de l'installation. De plus, une diminution du nombre des éléments de régulation entraîne aussi une augmentation de la précision et de la fiabilité de l'appareil 3.

**Revendications**

**1.** Dispositif de sélection pour fournir à un appareil (3) l'un de deux gaz, comprenant une première (11) et une deuxième (12) lignes d'alimentation respectivement d'un premier et d'un deuxième gaz, chaque ligne d'alimentation (11, 12) débouchant dans une ligne de purge (300, 302) respective, et une ligne de raccordement commune (304) des deux lignes d'alimentation (11, 12) à une ligne de fourniture commune (306) de gaz reliée à une entrée de l'appareil (3), une sortie de l'appareil (3) étant reliée à une ligne de purge additionnelle (308), un organe (310, 312, 314) de création d'une perte de charge étant disposé dans chaque ligne de purge (300, 302, 308), l'organe (310) de création d'une perte de charge dans la ligné de purge (300) associée à la première ligne d'alimentation (11) ainsi que l'organe (312) de création d'une perte de charge dans la ligne de purge additionnelle (308) étant des organes de régulation de débit, **caractérisé en ce que** l'organe (314) de création d'une perte de charge dans la ligne de purge (302) associée à la deuxième ligne d'alimentation (12) est un déverseur, et **en ce que** la ligne de fourniture commune (306) de gaz est uniquement reliée à la ligne de raccordement (304) d'une part, et à l'appareil (3) d'autre part.

**Claims**

**1.** Selection device for delivering one of two gases to an apparatus (3), comprising a first (11) and a second (11) line for respectively feeding a first and a second gas, each feed line (11, 12) emerging in a respective purge line (300, 302), and a common connection line (304) connecting the two feed lines (11, 12) to a common gas delivery line (306) connected to an inlet of the apparatus (3), an outlet of the apparatus (3) being connected to an additional purge line (308), an element (310, 312, 314) for creating a pressure drop being placed in each purge line (300, 302, 308), the element (310) for creating a pressure drop in the purge line (300) associated with the first feed line (11) as well as the element (312) for creating a pressure drop in the additional purge line (308) being flow-regulating elements, **characterized in that** the element (314) for creating a pressure drop in the purge line (302) associated with the second feed line (12) is a backpressure regulator and **in that** the common gas delivery line (306) is only connected, on the one hand, to the connection line (304) and, on the other hand, to the apparatus (3).

**Patentansprüche**

1. Auswahlvorrichtung zur Beschickung einer Apparatur (3) mit einem von zwei Gasen, enthaltend eine erste (11) und eine zweite (12) Leitung zur Zuführung eines ersten bzw. zweiten Gases, wobei jede Zufuhrleitung (11, 12) jeweils in eine Spülleitung (300, 302) mündet, und eine gemeinsame Leitung (304) zur Verbindung der beiden Zufuhrleitungen (11, 12) mit einer gemeinsamen Gasbeschickungsleitung (306), die mit einem Eingang der Apparatur (3) verbunden ist, einem mit einer zusätzlichen Spülleitung (308) verbundenen Ausgang der Apparatur (3), einem in jeder Spülleitung (300, 302, 308) angeordneten Element (310, 312, 314) zur Erzeugung eines Druckverlusts, wobei das Element (310) zur Erzeugung eines Druckverlusts in der mit der ersten Zufuhrleitung (11) assoziierten Spülleitung (300) sowie das Element (312) zur Erzeugnug eines Druckverlusts in der zusätzlichen Spülleitung (308) Durchflußregelemente darstellen, **dadurch gekennzeichnet, daß** es sich bei dem Element (314) zur Erzeugung eines Druckverlusts in der mit der zweiten Zufuhrleitung (12) assoziierten Spülleitung (302) um einen Rückdruckregler handelt und die gemeinsame Gasbeschickungsleitung (306) lediglich einerseits mit der Verbindungsleitung (304) und andererseits mit der Apparatur (3) verbunden ist.

FIG.1

EP 0 840 118 B1

FIG 3

FIG.2

FIG.4A

FIG.4B

EP 0 840 118 B1

FIG. 5